Europäisches Patentamt

European Patent Office

Office européen des brevets

(19)

(11) Publication number: **0 153 863**
**B1**

# (12) EUROPEAN PATENT SPECIFICATION

(45) Date of publication of patent specification: **01.03.89**

(51) Int. Cl.⁴: **C 03 C 8/02,** C 23 D 5/00

(21) Application number: **85301354.8**

(22) Date of filing: **28.02.85**

(54) **Bonding of bioactive glass coatings.**

(30) Priority: **01.03.84 US 584976**

(43) Date of publication of application:
**04.09.85 Bulletin 85/36**

(45) Publication of the grant of the patent:
**01.03.89 Bulletin 89/09**

(84) Designated Contracting States:
**AT CH DE FR GB IT LI SE**

(56) References cited:
**EP-A-0 013 906**
**EP-A-0 077 223**
**DE-A-3 248 649**

(73) Proprietor: **Pfizer Hospital Products Group, Inc.**
**235 East 42nd Street**
**New York New York 10017 (US)**

(72) Inventor: **Kucheria, Chattar Singh**
**187 Capstan Avenue**
**Mystic New London County (US)**

(74) Representative: **Wood, David John et al**
**Pfizer Limited Ramsgate Road**
**Sandwich Kent CT13 9NJ (GB)**

The file contains technical information
submitted after the application was filed and
not included in this specification

Courier Press, Leamington Spa, England.

## Description

The present invention relates to a method of bonding biologically active (hereafter called "bioactive") glass coatings to metal substrates, particularly medical prostheses, and to a metal substrate coated with bioactive glass.

Bioactive glasses for coating of metal substrates suitable for prostheses or surgical implants are known in the art, e.g. U.S. Patent 4,234,972. A method is disclosed allowing use of glasses and metals which do not have closely matched coefficients of thermal expansion.

Matching coefficients of thermal expansion are required when conventional coating methods are used requiring high firing temperatures. On subsequent cooling, thermomechanical stress in the glass layer is reduced when thermal expansion coefficients match so avoiding cracks in the glass coating.

EP—A—0077223 discloses a prosthesis consisting of a metal substrate coated with a vitreous enamel containing cobalt oxide as a constituent of the enamel. The vitreous enamel is applied to the metal substrate and fired at a temperature lower than 800°C.

DE—A—3248649 discloses a metal substrate coated with a bioactive glass. EP—A—0013906 discloses a glaze frit for a metal substrate containing cobalt or nickel oxide. "Emaillierung", A. H. Dietzel, Wissenshaftliche Grundlagen und Grundzuge der Technologie, 1981, pp. 119—120, describes an enamel for sheet steel containing cobalt or nickel oxide.

It is an object of the invention to increase the bond strength between the glass and the metal by developing a chemical bond between the glass and the metal on firing the glass-coated metal substrate at high temperature. In view of the high firing temperatures matching thermal expansion coefficients are essential.

The invention is concerned with a method of bonding a bioactive glass coating to a metal substrate characterised by applying to a metal substrate a slurry containing a mixture of bioactive glass and at least one metal oxide selected from cobaltic oxide and cobaltous oxide, and firing the coated substrate at a temperature of at least about 730°C, the bioactive glass having substantially the same coefficient of expansion as said metal substrate.

The metal oxide used in the invention is cobaltic oxide ($Co_2O_3$) or cobaltous oxide (CoO) or an equimolar mixture therof ($Co_3O_4$). The metal oxide is generally applied in an amount of about 0.2—3.0% based on the dry bioactive glass.

According to the invention, any bioactive glass may be used having the same coefficient of expansion as the metal substrate to which it is applied. Bioactive glasses on reaction with body fluids form a series of surface reactive films bonding living tissue such as bone to the glass. Suitable bioactive glasses include those having the following composition by weight:

| | |
|---|---|
| $SiO_2$ | 40—60% |
| $Na_2O$ | 10—32% |
| CaO | 10—32% |
| $P_2O_5$ | 0—12% |
| $CaF_2$ | 0—18% |
| $B_2O_3$ | 0—20%, |

The above ingredients totalling 100%.

Specific bioactive glasses include the following:

| | |
|---|---|
| $SiO_2$ | 43—48% |
| $Na_2O$ | 18—21% |
| CaO | 19—22% |
| $B_2O_3$ | 4—15% |
| $P_2O_5$ | 4—10% |

the above ingredients totalling 100%. More specifically, the following bioactive glasses are included:

(1)

| Composition | % by weight |
|---|---|
| $SiO_2$ | 44.65 |
| $Na_2O$ | 20.01 |
| CaO | 19.59 |
| $B_2O_3$ | 10.30 |
| $P_2O_5$ | 5.45 |

The thermal expansion coefficient of this composition is $13.43 \times 10^{-6}/°C$.

(2)

| Composition | % by weight |
|---|---|
| $SiO_2$ | 43.83 |
| $Na_2O$ | 18.41 |
| CaO | 21.63 |
| $B_2O_3$ | 10.11 |
| $P_2O_5$ | 6.02 |

The thermal expansion coefficient of this composition is $13.31 \times 10^{-6}/°C$.

(3)

| Composition | % by weight |
|---|---|
| $SiO_2$ | 47.45 |
| $Na_2O$ | 19.48 |
| CaO | 21.60 |
| $B_2O_3$ | 5.45 |
| $P_2O_5$ | 6.02 |

The thermal expansion coefficient of this composition is $13.12 \times 10^{-6}/°C$.

Suitable metals for the metal substrate include any metal from which an artificial prosthesis may be manufactured. Examples of suitable metals include steels such as surgical stainless steel and carbon steel, cobalt-chrome alloys such as cobalt-chrome-molybdenum alloys, titanium, titanium alloys, noble metals such as platinum, and noble metal alloys. Vitallium (trademark) alloy is a preferred alloy and consists of:

| Element | % by weight |
|---|---|
| Carbon | 0.25 |
| Silicon | 0.75 |
| Manganese | 0.70 |
| Chromium | 28.00 |
| Molybdenum | 5.50 |
| Cobalt | 64.80 |

The thermal expansion coefficient of cast Vitallium is $13.21 \times 10^{-6}/°C$ substantially matching those of the above three compositions.

The bioactive glass compositions are made by usual methods for making glasses involving mixing of the ingredients, melting and then cooling.

The bioactive glass composition is admixed in a slurry with cobaltic oxide, cobaltous oxide, or suitable mixtures thereof. The mixing is conveniently achieved by preparing a slurry of the glass and adding the metal oxide to the slurry. The slurry typically contains small particles of mean size of about 0.0065 mm of the bioactive glass, a liquid such as ethanol or acetone, and an emulsifier.

The admixture of glass and metal oxide is applied to the metal substrate by conventional methods. Usually, the application is by spray-coating at room temperature, with a slurry such as described above.

The metal substrate is often treated to increase adherence of the glass coating to the substrate. Suitable treatment includes oxidation and roughening of the substrate surface, e.g. as described in U.S. Patent 4,159,358.

The metal substrate surface is usually cleaned, preferably ultrasonically in methanol, and dried such as by gentle blow-drying with a particle-free aerosol. After optional oxidation or roughening, the glass-metal oxide slurry is applied by spray-coating. Suitably, an airbrush is used at a distance of 3.5 inches (8.9 cm) at 10 psi (73 KPa) and variable angles to ensure uniform coating.

After application of the glass-metal oxide slurry, the alloy is allowed to dry before firing. The firing is at a temperature of at least 730°C, and usually not higher than 760°C. Below 730°C and above 760°C, the bond between the substrate and the coating tends to be less satisfactory.

The adherence of the glass to the metal is tested by holding one end of a glass coated metal strip in a vice and the other end of the strip in a vice grip. On twisting the strip by about 10°, the strip and the coating are examined visually and microscopically. In the test, the glass coating will break due to the mechanical stress caused by the twisting action. Depending on the strength of the glass metal bond, the glass coating may become entirely detached from the metal strip evidencing no glass metal bond, or the glass coating may partly or entirely adhere to the metal strip even though the glass coating itself breaks under the stress of the test. This is a very severe test.

The following examples illustrate the invention.

Example 1 (Comparative)

A slurry was prepared containing the following:

| | |
|---|---|
| Ethanol (95%) | 112.00 g |
| Butvar® B-76 resin | 1.67 g |
| Triton® N-150 emulsifier | 0.83 g |
| Bioactive glass (2) above | 55.00 g |

Butvar B-76 resin is polyvinylbutyryl resin.

Triton N-150 emulsifier is liquid nonylphenoxy polyethoxy ethanol.

The bioactive glass is glass composition (2) described above. The glass particles had a mean particle size of 0.0053 mm.

After mixing for 24 hours, the slurry was spray coated at ambient temperature on a strip of Vitallium that had first been roughened, cleaned and then oxidized. The coating was dried under ambient conditions and then fired at up to 732°C in air for one minute.

The strip was tested for adherence by the above very severe adherence test. The glass coating separated in a layer leaving behind a shiny white metal surface without any glass remaining adhered to the metal. Thus, at least in this severe test, the glass coating had poor adherence to the metal.

Example 2

The glass slurry (20 g) prepared in Example 1 was mixed with 0.15 g cobaltic oxide $(Co_2O_3)$ resulting in a net cobaltic oxide content of 2.31% based on the weight of the dry glass. After mixing for 24 hours, the slurry was spray coated at ambient temperature on pre-roughened preoxidized Vitallium strips. The strips were dried and fired as described in Example 1. The formed glass coated strips were tested for adherence. As a result of the test, the glass coating fractured. However, the glass coating fragments remained adhered to the metallic strip. Addition of $Co_2O_3$ thus increased the adherence of glass to metal.

Example 3

A slurry was prepared containing the following:

| | |
|---|---|
| Ethanol (95%) | 150.0 g |
| Butvar B-76 resin | 2.24 g |
| Triton N-150 emulsifier | 1.11 g |
| Bioactive glass (2) above | 75.0 g |
| Mixture of cobaltous oxide and cobaltic oxide $(Co_3O_4)$ | 0.75 g |

The bioactive glass had a mean particle size of 0.0056 mm and contained 1% cobalt oxide on a dry glass basis. A preoxidized Vitallium strip was coated with the slurry as described in Example 2 and tested for adherence. The glass coating broke. Some of the glass coating became detached from the strip so adherence was not as good as in Example 2.

Example 4

A glass slurry was prepared as in Example 3 except that 0.75 g $Co_2O_3$ was substituted for 0.75 g $Co_3O_4$.

The cobalt oxide content of the slurry remained 1% based on the dry glass.

The glass coated strips were prepared in a similar manner as in Example 1 and tested for adherence. The glass coating broke and the coating fragments remained adhered to the metal strip showing very good adherence. Fewer and smaller bubbles were observed than in Example 2.

Example 5

Two parts of the slurry of Example 3 were mixed with one part of the slurry of Example 4 for 24 hours. The cobalt oxide content of the slurry was 0.67% $Co_3O_4$ and 0.33% $Co_2O_3$ based on the dry glass.

Glass coated strips were prepared as in Example 1 and tested for adherence. The glass coating broke and fragments thereof remained adhered to the metal strip showing very good adherence. The glass coating contained very few bubbles.

**Claims**

1. A method of bonding a bioactive glass coating to a metal substrate characterized by applying to a metal substrate a slurry containing a mixture of bioactive glass and at least one metal oxide selected from cobaltic oxide and cobaltous oxide, and firing the coated substrate at a temperature of at least about 730°C, the bioactive glass having substantially the same coefficient of expansion as the metal substrate.

2. A method according to claim 1 characterized in that the metal oxide or oxides are applied in a total amount of from 0.2—3.0% by weight based on the weight of the dry bioactive glass.

3. A method according to claim 1 or 2, characterized in that the bioactive glass is applied by spray-coating.

4. A method according to claim 1, 2 or 3, characterized in that the metal substrate is dried after application of said bioactive glass.

5. A method according to any one of claims 1 to 4, characterized in that the metal substrate is a cobalt-chromium-molybdenum alloy.

6. A method according to claim 1, characterized in that the mixture of bioactive glass and metal oxide is in the form of an ethanol slurry.

7. A method according to any one of the preceding claims, wherein the firing is carried out at a temperature of from 730° to 760°C.

8. A method as claimed in any one of claims 1 to 7, wherein the bioactive glass composition consists of the following ingredients in percentages by weight based on the total weight of the glass:—

| | |
|---|---|
| $SiO_2$ | 43—48 |
| $Na_2O$ | 18—21 |
| CaO | 19—22 |
| $B_2O_3$ | 4—15 and |
| $P_2O_5$ | 4—10, |

the total of said ingredients being 100%.

**Patentansprüche**

1. Verfahren zum Binden einer bioaktiven Glasbeschichtung an ein Metallsubstrat, dadurch gekennzeichnet, daß auf ein Metallsubstrat eine eine Mischung von bioaktivem Glas und mindestens einem Metalloxid ausgewählt aus Kobalt(III)-oxid und Kobalt(II)-oxid enthaltende Aufschlämmung aufgebracht und das beschichtete Substrat bei einer Temperatur von mindestens etwa 730°C gebrannt wird, wobei das bioaktive Glas im wesentlichen den gleichen Expansionskoeffizienten wie das Metallsubstrat aufweist.

2. Verfahren nach Anspruch 1, dadurch gekennzeichnet, daß das Metalloxid oder die Metalloxide in einer Gesamtmenge von 0,2 bis 3,0 Gew.-%, bezogen auf das Gewicht des trockenen bioaktiven Glases, aufgebracht werden.

3. Verfahren nach Anspruch 1, dadurch gekennzeichnet, daß das bioaktive Glas durch Sprühbeschichten aufgebracht wird.

4. Verfahren nach Anspruch 1, 2 oder 3, dadurch gekennzeichnet, daß das Metallsubstrat nach Aufbringen des bioaktiven Glases getrocknet wird.

5. Verfahren nach einem der Ansprüche 1 bis 4, dadurch gekennzeichnet, daß das Metallsubstrat eine Kobalt-Chrom-Molybdänlegierung ist.

6. Verfahren nach Anspruch 1, dadurch gekennzeichnet, daß die Mischung von bioaktivem Glas und Metalloxid in Form einer Äthanolaufschlämmung ist.

7. Verfahren nach einem der vorhergehenden Ansprüche, worin das Brennen bei einer Temperatur von 730 bis 760°C durchgeführt wird.

8. Verfahren, wie in einem der Ansprüche 1 bis 7 beansprucht, worin die bioaktive Glaszusammensetzung aus folgenden Bestandteilen in Gew.%, bezogen auf das Gesamtgewicht des Glases, besteht:

| | |
|---|---|
| $SiO_2$ | 43 bis 48 |
| $Na_2O$ | 18 bis 21 |
| CaO | 19 bis 22 |
| $B_2O_3$ | 4 bis 15 |
| $P_2O_5$ | 4 bis 10, |

wobei die obigen Bestandteile insgesamt 100% ausmachen.

**Revendications**

1. Procédé de liaison d'un revêtement de verre biologiquement actif à un substrat métallique, caractérisé en ce qu'il consiste à appliquer à un substrat métallique une suspension contenant un mélange de verre biologiquement actif et d'au moins un oxyde métallique choisi entre l'oxyde cobaltique et l'oxyde cobalteux, et à chauffer le substrat revêtu à une température d'au moins environ 730°C, le verre biologiquement actif ayant pratiquement le même coefficient de dilatation que le substrat métallique.

2. Procédé suivant la revendication 1, caractérisé en ce que le ou les oxydes métalliques sont appliqués en une quantité totale de 0,2 à 3,0% en poids, sur la base du poids du verre sec biologiquement actif.

3. Procédé suivant la revendication 1 ou 2, caractérisé en ce que le verre biologiquement actif est appliqué par revêtement par pulvérisation.

4. Procédé suivant la revendication 1, 2 ou 3, caractérisé en ce que le substrat métallique est séché après application du verre biologiquement actif.

5. Procédé suivant l'une quelconque des revendications 1 à 4, caractérisé en ce que le substrat métallique est un alliage cobalt-chrome-molybdène.

6. Procédé suivant la revendication 1, caractérisé en ce que le mélange de verre biologiquement actif et d'oxyde métallique est sous forme d'une suspension éthanolique.

7. Procédé suivant l'une quelconque des revendications précédentes, dans lequel le chauffage est effectué à une température de 730° à 760°C.

8. Procédé suivant l'une quelconque des revendications 1 à 7, dans lequel la composition de verre biologiquement actif comprend les ingrédients suivants, en pourcentages en poids, sur la base du poids total du verre:

| | | |
|---|---|---|
| $SiO_2$ | 43—48 | |
| $Na_2O$ | 18—21 | |
| CaO | 19—22 | |
| $B_2O_3$ | 4—15 | et |
| $P_2O_5$ | 4—10, | |

la quantité totale desdits ingrédients étant égale à 100%.